# EUROPEAN PATENT SPECIFICATION

(11) **EP 1 951 345 B1**
(45) Date of publication and mention of the grant of the patent: **20.11.2013**
(21) Application number: 06805538.3
(22) Date of filing: 27.10.2006
(51) Int. Cl.: A61M 5/44

(54) **PORTABLE DEVICE FOR HEATING AND ADMINISTERING INTRAVENOUS FLUIDS**
TRAGBARE VORRICHTUNG ZUR ERWÄRMUNG UND VERABREICHUNG VON INTRAVENÖSEN FLÜSSIGKEITEN
DISPOSITIF PORTABLE POUR CHAUFFER ET ADMINISTRER DES FLUIDES INTRAVEINEUX

(30) Priority: 27.10.2005 EP 05077469
(43) Date of publication of application: 06.08.2008
(73) Proprietor: Veinux ApS, 8200 Aarhus N (DK)
(72) Inventor: HANSEN, Jan, Erik, Vest, DK-8000 Arhus C (DK)
(74) Representative: Schmidt, Jens Joergen
(86) International application number: PCT/DK2006/000598
(87) International publication number: WO 2007/048414

(56) References cited:
- US-A1- 2002 156 451
- US-A1- 2003 225 396

## Description

### FIELD OF INVENTION

The present invention relates to a system for heating and administering a flow of intravenous fluid and to an assembly for a system for connecting a source of intravenous fluid to a patient.

### BACKGROUND

Army medics, paramedics, mountain rescue teams and other types of rescue personnel are often in need for administering intravenous fluids to critically wounded persons. However, when these intravenous fluids are carried in the field the temperature of these fluids is often substantially lower than the body temperature of the patient. The injection of cold intravenous fluids in to a vein of a patient presents a substantial risk for hypothermia, which poses a threat to the patient's life. Therefore there is a need for a portable device to heat the intravenous fluid to a temperature closer to the body temperature before it enters the patient's veins.

WO 01/62194 A1 discloses a portable disposable intravenous fluid warming system. The system comprises a battery powered unit with an integrated heating element having an inlet and an outlet for intravenous fluids, sized to fit standard intravenous line connectors. The inlet of the unit is connected to an intravenous bag and the outlet is connected to an intravenous catheter via standard intravenous tubes.

US 6,236,809 discloses a system for heating intravenous fluids comprising a disposable heat exchanger with an inlet and an outlet for intravenous fluids and a portable battery powered unit including a heating element and temperature controller. The inlet of the disposable heat exchanger comprises a fitting for connection to a source of intravenous fluid via a first length of tubing, and the outlet of the disposable heat exchanger likewise comprises a fitting for connection to an intravenous catheter via a second length of tubing.

US 5,250,032 disclose yet another system for warming intravenous fluids. The system comprises a battery powered heating element and a control circuit placed in housing mounted on the patients arm. An elongated portion of a standard intravenous tube is inserted in a channel in the heating element, whereby a flow of intravenous fluid in the intravenous tube is heated.

US 3,908,652 disclose a medical infusion apparatus having an infusion flask exchangeably fastened to a casing, an infusion tube provided with a drip chamber, an electrical control system, and a drip valve or an infusion pump. A heat radiation source may be incorporated in the apparatus in order to heat the infusion liquid on its path from the flask to the infusion tube.

US 2002/0156451 A1, WO 96/11037 and EP 1 066 844 A1 all discloses heating means for heating of intravenous fluids by means of electrical resistor heaters arranged within the intravenous tube.

US 4,684,367 discloses a system for intravenous delivery of fluids to patients, which includes means to apply a pressure to the bag holding the fluid and having fluid heating means, flow measuring means and flow regulating means that the flow of fluid passes before reaching the patient.

US 2003/0225396 A1 discloses an infusion system for intravenous delivery of fluid, having a portable unit including a pump assembly, in particular a roller pump having an opening for receiving a length of a flexibly intravenous tube the outer surface of which is manipulated by the pump in order to pump the fluid. The portable unit further comprises rechargeable cells for providing electrical power to heat intravenous fluid flowing in the intravenous tube, and the intravenous tube may be a part of a disposable fluid containment system including a pump cartridge containing a drip chamber and the pump chamber the intravenous tubing, outlet infusion tubing and a heater cartridge.

It is an object of the present invention to provide a simple system to provide a heated flow of intravenous fluid prior to its entry into the vein of a patient, and to heat said flow of intravenous fluid sufficiently to avoid hypothermia to the body of a receiving patient.

Additionally, it is an object of the present invention to provide a hygienic system for providing and heating a flow of intravenous fluid between a source of intravenous fluid and an intravenous catheter.

It is also an object of the present invention to provide a system for accurately administering a flow of intravenous fluid to a patient.

### BRIEF DESCRIPTION OF THE PRESENT INVENTION

With the present invention is provided a portable unit, in which an electrical energy source for heating of the fluid is provided integrally in the same housing as a pump for controlling the flow of the intravenous fluid by manipulating the outer surface of the intravenous tube. Thus, a simple system for administering a heated intravenous fluid to a under field conditions patient is provided, where the advantages of heating the fluid is combined with the provision of a pump that enables administration of a predefined flow rate of the fluid regardless of vertical position of the container of the intravenous fluid with respect to the patient. When combined with an intravenous tube provided enclosed in a sterile packaging and comprising heating means integrated into the tube for raising the temperature of the intravenous fluid flow, a hygienic system is provided where the sterility of the path of the intravenous fluid flow is easily obtained and preserved, as the intravenous tube can be connected to the container holding the intravenous fluid and to the patient and the portable unit can be connected to the tube for providing heating power and a flow driving pump without interfering with the sterile path of the fluid. Thus, a single infrangible path between the source of intravenous fluid and the intravenous catheter is safely provided.

The present invention as defined by claim 1 relates to a portable unit for providing energy to heat intravenous fluids, the unit comprising a housing wherein is arranged an elongated opening to receive a length of a flexible intravenous tube for connecting a source of intravenous fluid to an intravenous catheter, an energy source comprising one or more rechargeable cells arranged to provide electrical power to heat intravenous fluid flowing in the intravenous tube, and a pump for controlling the flow rate of intravenous fluid in the intravenous tube, the pump operating by manipulating the outer surface of the length of an intravenous tube received in said elongated opening by means of a single piston that during operation alternately compresses and releases the compress of the piece of flexible and elastic length of intravenous tube between two one-way valves comprised in the intravenous tube, and in that the housing further including mounting means for fastening the unit to a patient receiving the intravenous fluids.

The present invention as defined by claim 14 also relates to a system for heating a flow of intravenous fluid comprising the portable unit and an intravenous tube enclosed in a sterile packaging and having a liquid inlet and a liquid outlet for connecting a source of intravenous fluid to an intravenous catheter, said intravenous tube comprising heating means integrated therein for raising the temperature of the intravenous flow in the tube two one-way valves, and connection means for connecting said heating means with an external energy source.

The intravenous tube is preferably delivered sterile and enclosed in a sterile packaging until time of use. Hence, the risk of giving the patient an infection is substantially reduced.

Thus, the present invention relates to the portable unit as well as to a system comprising the portable unit and an intravenous tube including the use hereof.

Hereby, a simple portable system is provided which requires only two connections to external units as compared to most known systems which require multiple connections between several external units, i.e. that a separate heating element is not required by the provision of the system according to present invention to be inserted in between two lengths of intravenous tubing.

The simplicity of the present invention further eases the process of administering intravenous fluids to critical wounded persons and minimises the risk for rescue personnel under stress, e.g. an army medic at the front line or a paramedic at a large scene of accident, form making mistakes during the infusion of heated intravenous fluids, in that a separate heating element is not required to be connected to the flow path from the source of intravenous fluid to the patient.

Further the single infrangible path between a source of intravenous fluid and an intravenous catheter provides a highly hygienic system, because the number of connections wherein the flow of intravenous fluid can be contaminated is limited to a minimum as compared to other systems with multiple connections to external units.

It is advantages that the housing of the portable unit includes mounting means for fastening the unit to a patient receiving the intravenous fluids. In a particular embodiment, the portable unit is formed as a flexible wristband and the mounting means includes straps of the like to fasten the portable unit around the arm of the patient to receive the intravenous fluid.

The energy source is preferably able to supply sufficient power to raise the temperature of the intravenous fluid with an amount between 20 °C and 30 °C, preferably between 23 °C and 27 °C at a flow rate of 100 ml/min.

The energy source may be connected to a heating element in the portable unit, which then is in contact with the outer surface of a part of the intravenous tube, preferably the part received in the elongated opening of the portable unit, but in a preferred embodiment of the present invention, the energy source comprises means for supplying electrical power to heating means arranged externally to the unit, such as within the intravenous tube. The means for supplying electrical power are advantageously arranged within or adjacent to the elongated opening of the housing, in particularly so that the electrical contact between said means and corresponding electrical terminals on the tube is established when the intravenous tube is placed correctly in the elongated opening in the portable unit.

In the case where the heating means are integrated into the intravenous tube, it is advantageous that the surface area in contact with the intravenous fluid of said heating means comprises an area of at least 20 cm², preferably at least 30 cm² and most preferred at least 40 cm² so as to ensure that the heating of the fluid is performed gentle. By having a substantially large heating area in contact with the intravenous fluid, a low contact temperature between the heating means and the intravenous fluid is obtained, thereby providing a gentle heating of the intravenous fluid.

In another aspect of the present invention said integrated heating means comprises one or more heating wires inlaid in the enclosure of the intravenous tube. The intravenous fluid is heated while flowing through the enclosure of the intravenous tube, thereby providing a large area of the heating means which is in contact with the intravenous fluid. This means that the intravenous fluid can be gently heated, thus preventing damage to fragile intravenous fluids such as blood.

In yet another aspect of the present invention said integrated heating means comprising heating wires encased in the wall of the intravenous tube. Hence, the heating wires are protected within the wall of the intravenous tube, thereby providing heating means that is resistant for use in harsh environments, such as when the intravenous tube are carried in the field in a densely packed bag which is exposed to large temperature gradients, shocks and blows.

The heating means extends preferably a substantial portion of the tube length preferably between 75 cm and 200 cm of the tube length such as between 125 cm and 175 cm of the tube length. Hence, it is assured that the flow of intravenous fluid is gently heated without being damaged, and that the heat loss through the tube wall is reduced to a minimum.

In a preferred embodiment of the present invention, the heating element extends out from the coupling of the tube to the container for the fluid and into the container, so at to keep the content of the container above freezing temperature during operation and thereby prevent the formation of ice crystals that may damage the fluid, e.g. in case of blood, or may cause clogging of the flow path.

With the pump in the portable unit, a flow of intravenous fluid can be provided without the aid of gravity and the amount of intravenous fluid administered to a patient can easily be controlled by the rescue personnel. The pump may in particular be a peristaltic pump, i.e. a pump operating by manipulating the outer surface of a flexible length of the intravenous tube without having parts of the pump in directly contact with the fluid itself. Thereby, the flow of intravenous fluid delivered to a patient can be controlled without undermining the sterile path of the intravenous fluid from a source of intravenous fluid to a patient. Further the amount of intravenous fluid delivered to the patient can easily be established and controlled. According to the invention, the intravenous tube comprises two one-way valves and the pump comprises a single piston that during operation alternately compresses and releases the compress of the piece of flexible and elastic length of intravenous tube between the two valves and situated in the elongated opening of the portable unit.

The portable unit may further comprise control means for controlling the operation of at least one element of the unit, in particular the supply of power to the heating of the fluid and/or the pump, in response to at least one control input from means for providing a measure of at least one physical property of the flow of intravenous fluid, such as in particular the temperature of the fluid and/or the flow rate.

In one embodiment, said means for providing a measure of at least one physical property of the intravenous fluid comprises temperature measure means for measuring the temperature of the flow of fluid in the intravenous tube and providing an output accordingly to the control means. The temperature measuring means are preferably arranged within or adjacent to the elongated opening of the housing.

Additionally or alternatively, said means for providing a measure of at least one physical property of the intravenous fluid comprises means for providing a measure of the flow rate of fluid in the intravenous tube and providing an input accordingly to the control means. The means for providing a measure of the flow rate of fluid in the intravenous tube may further comprise alarm means for providing an alarm signal when the source of intravenous fluid is empty and/or comprise alarm means for providing an alarm signal in case the flow of intravenous fluid is interrupted.

It is preferred that the operation of the pump and/or the supply of energy from the energy source to heat intravenous fluid flowing in the intravenous tube are controlled so as to limit the temperature of the flow of intravenous fluid to a temperature interval between 35°C and 40°C such as between 36°C and 38°C.

### BRIEF DESCRIPTION OF THE DRAWINGS

The invention will be described in the following with reference to the drawings of which
Fig. 1 illustrates a system for providing and heating a flow of intravenous fluid according to a preferred embodiment of the present invention, and
Fig. 2a to 2f illustrates cross sectional views of different embodiments of intravenous tubes according to the present invention.

### DETAILED DESCRIPTION OF THE PRESENT INVENTION

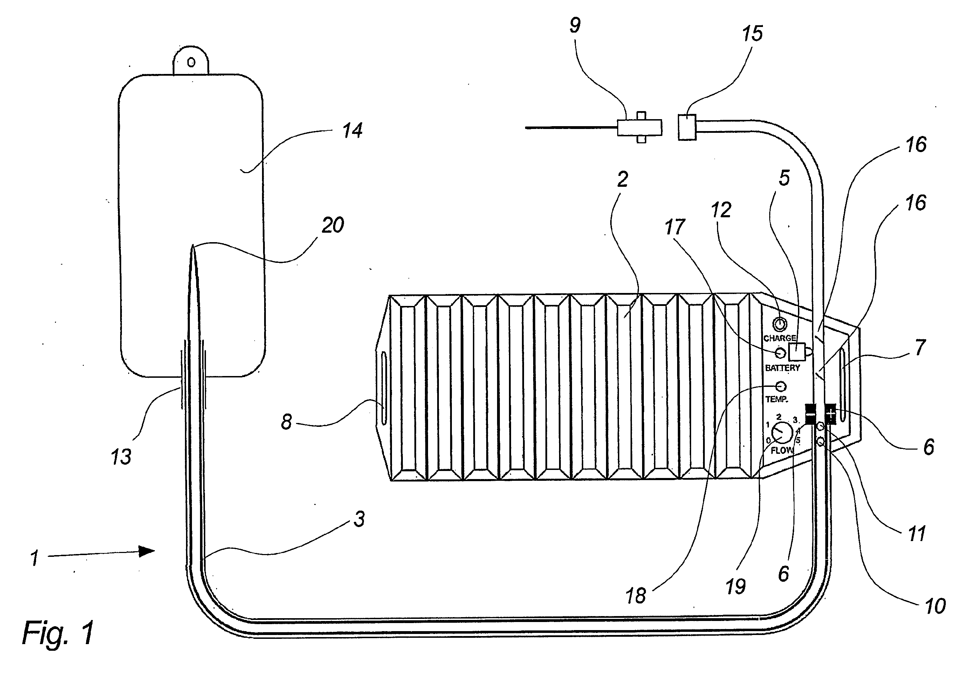

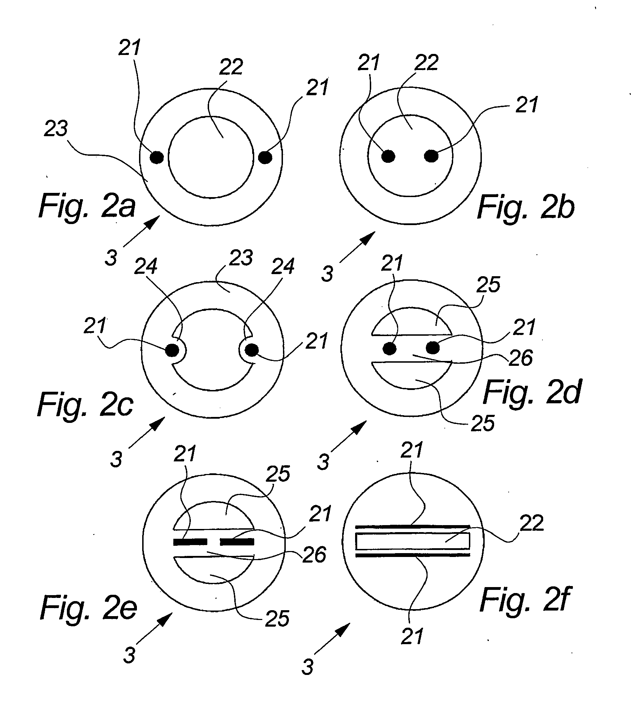

Fig. 1 illustrates a system 1 for heating a flow of intravenous fluid according to a preferred embodiment of the present invention. The system 1 comprises two separate main parts, a portable unit 2 in form of a wristband and an intravenous tube 3 with electrical heating wires (examples shown in Figs. 2a to 2f) integrated therein. A length of the tube 3 is temporarily arranged in an elongated opening 4 of the wristband 2 when the system is about to be used. The wristband may be used a multitude of times while the intravenous tube 3 is for single use only. A peristaltic pump 5 is integrated in the wristband and operates on the part of the intravenous tube 3 housed in the elongated opening 4 for an accurate control of the intravenous fluid delivered to a patient.

The portable unit 2 comprises an energy source in form of a plurality of rechargeable electrical batteries arranged along the circumference of the wristband. The portable unit 2 further comprises a socket sized to fit a corresponding connector 6 mounted on the intravenous tube 3. By forming the portable unit 2 as a wristband with openings 7, 8 for mounting of a strip to fasten the wristband to an arm of a patient the intravenous tube 3 can be fixated to the wrist of a patient thereby preventing the intravenous catheter 9 from being pulled out of the vein of the patient during transport.

The portable unit 2 further comprises a temperature controller in order to control the heating of the intravenous fluid. Near the connector 6 a built-in temperature sensor 10, e.g. an RTD, provides an input to the temperature controller according to the actual temperature of the intravenous fluid. Hereby, the system is able to prevent that the intravenous fluid is heated above body temperature. As a further safety precaution the portable unit 2 comprises a thermal fuse 11 arranged near the socket 6 in thermal contact with the intravenous fluid e.g. via the connector 6, to cut off the power to the electrical heating elements in case the temperature control system fails. Alternatively, the temperature sensor 10 could also be arranged in the portable unit 2 in thermal contact with the intravenous fluid e.g. via the connector 6. The connector 6 is preferably made of metal or a heat conductive plastic in order to provide the thermal fuse 11 and/or the temperature sensor 10 with an accurate measure of the temperature of the intravenous fluid.

The system is capable of raising the temperature of the intravenous fluid 25°C above its initial temperature at a flow rate of 100 ml/min. If the temperature of the intravenous fluid reaches body temperature (approximately 37°C) the temperature controller cuts off the power to the electrical heating wires until the temperature of the intravenous fluid is below body temperature.

The portable unit 2 further comprises a second socket 12 for connecting the unit 2 with a utility grid or the electrical system of a motorised vehicle, thereby providing means for supplying the electrical heating wires with an alternative source of electricity and/or for recharging the rechargeable batteries, when the external unit is connected to an alternative source of electricity.

The intravenous tube 3 comprises a liquid inlet releasably connected to an intravenous bag 14 and a liquid outlet 15 for releasably connecting the intravenous tube 3 to an intravenous catheter 9 to be inserted into a vein of a patient. In order to heat a flow of intravenous fluid flowing through the intravenous tube 3, a pair of electrical heating wires (examples shown in a cross sectional view in Figs. 2a to 2f) preferably of stainless steel and integrated within the intravenous tube 3 extends inside the tube 3. The electrical heating wires are supplied with electrical power from the portable unit 2 through the connector 6. The heating wires extend 20 into the bag 14 containing the fluid to prevent it from freezing during operation.

A length of intravenous tubing 3 without integrated heating wires extends inside the elongated opening 4 and is equipped with two one-way valves 16, and the peristaltic pump 5 operates on the tube 3 between these valves 16 in order to accurately control the flow of intravenous fluid from the intravenous bag 9 to the intravenous catheter.

The portable unit 2 further comprises a light diode 17 for indication of the charging condition of the rechargeable batteries, a light diode for indication of the fluid temperature being within the correct range and a control knob 19 for manual adjustment of the flow rate.

When the tube 3 is placed in the elongated opening and the connector 6 of the intravenous 3 tube is inserted into the socket of the portable unit 2, electrical power is automatically transferred from batteries in the portable unit 2 to the electrical heating wires in the intravenous tube 3. The electrical heating wires with an outer diameter of 0.5 millimetres extend approximately 150 cm from the connector 6 to the part 20 extending into the bag 14. Thereby, a gentle heating of the flow of intravenous fluid is assured and damage to fragile intravenous fluids such as blood is prevented.

The peristaltic pump 5, which is also powered by the rechargeable batteries of the portable unit 2, provides a controlled flow of intravenous fluid. Hereby the rescue personnel can administrate the amount of intravenous fluid delivered to a patient according to instructions from a doctor situated at remote location from the scene of accident. The doctor could e.g. be situated at a hospital and send his instructions to the rescue personnel treating a patient in the field via radio or telephone. Additionally the peristaltic pump 5 is capable of measuring the amount of intravenous fluid delivered to a receiving patient, and providing an alarm signal when the source 14 of intravenous fluid is empty. The peristaltic pump 5 further comprises flow measuring means in order to detect if the flow of intravenous fluid is interrupted, thereby providing an alarm signal if the flow of intravenous fluid is interrupted, e.g. if the intravenous tube 3 is twisted or pinched during transport of the patient.

The invention has been exemplified above with reference to specific examples of a system for heating intravenous fluids. However, it should be understood that the invention is not limited to the particular examples described above but may be designed and altered in a multitude of varieties within the scope of the invention as specified in the claims.

Figs. 2a to 2f illustrates cross sectional views of different embodiments of the intravenous tube 3 with integrated electrical heating wires 21. Although the shape of the electrical heating wires 21 differs in the different embodiments shown in Figs. 2a to 2f, it is common to all embodiments that the electrical heating wires either extends into the bag 14 of fluid or are short circuited by a metal ring. The embodiments shown in the figures are only meant as examples, so other embodiments with other configurations of the heating wires 21 are also within the scope of the present invention.

Fig. 2a shows a cross sectional view of a first embodiment of the intravenous tube 3 according to the present invention. The intravenous fluid flows through a circular inner portion 22, and is heated via circular electrical heating wires 21 encased within the wall 23 of the intravenous tube 3.

Fig. 2b shows a cross sectional view of a second embodiment of the intravenous tube 3 according to present invention. The intravenous fluid flows through a circular inner portion 22, and is heated via circular heating wires 21 placed directly inside the circular portion 22 of the intravenous tube 3.

Fig 2c shows a cross sectional view of a third embodiment of the intravenous tube 3 according to present invention. The intravenous fluid flows through the circular inner portion 22 of the intravenous tube 3, and is heated by circular electrical heating wires 21 encased within internal thickenings 24 of the inner portion of the wall 23 of the intravenous tube 3.

Fig 2d shows a cross sectional view of a fourth embodiment of the intravenous tube 3 according to present invention. The intravenous fluid flows within two semi circular inner portions 25 of the intravenous tube 3 divided by an inner wall 26. The intravenous fluid is heated by circular electrical heating wires 21 encased within the inner wall 26.

Fig 2e shows a cross sectional view of a fifth embodiment of the intravenous tube 3 according to present invention. The intravenous fluid flows within two semi circular inner portions 25 of the intravenous tube 3 divided by an inner wall 26. The intravenous fluid is heated by rectangular electrical heating wires 21 encased within the inner wall 26. The rectangular shape of the electrical heating wires 21 provides a larger surface area from which heat can be transferred to the intravenous fluid, as compared to the circular electrical heating wires 21.

Fig 2f shows a cross sectional view of a preferred embodiment of the intravenous tube 3 according to present invention. The intravenous fluid flown through a rectangular inner portion 27 of the intravenous tube, and is heated by rectangular shaped heating wires 21 arranged along the longer sides of the rectangle. The electrical heating wires 21 could advantageously be two sheets of foil. Hereby the intravenous fluid flows past heating wires with a large surface area. Hence, an effective transfer of heat between the heating wires 21 and the intravenous fluid is provided, thereby minimising the heat loss from the intravenous fluid. Further the ratio between the area of the electrical heating element and the power delivered to the heating element can be substantially lowered, thus precluding a meltdown of the heating element. Another advantage of the low power to area ratio is a low contact temperature between the heating wires and the intravenous fluid, thereby providing a gentle heating of the intravenous fluid.

## Claims

1. A portable unit (2) for providing energy to heat intravenous fluids and for administration of said fluids, the unit comprising a housing wherein is arranged
an elongated opening (4) to receive a length of a flexible elastic intravenous tube (3) for connecting a source (14) of intravenous fluid to an intravenous catheter (9),
a flexible elastic intravenous tube (3),
an energy source comprising one or more rechargeable cells arranged to provide electrical power to heat intravenous fluid flowing in the intravenous tube (3), and
a pump (5) for controlling the flow rate of intravenous fluid in the intravenous tube (3), the pump (5) operating by manipulating an outer surface of said length of the flexible intravenous tube (3),
**characterised in that**
the pump (5) operates by manipulating the outer surface of said flexible length of the intravenous tube (3) received in said elongated opening (4) by means of a single piston that during operation alternately compresses and releases the outer surface of the flexible and elastic length of intravenous tube (3) between two one-way valves (16) comprised in the intravenous tube (3), and **in that** the housing further including mounting means (7, 8) for fastening the unit to a patient receiving the intravenous fluids.

2. A portable unit (2) according to claim 1, wherein said energy source is able to supply sufficient power to raise the temperature of the intravenous fluid with an amount between 20 °C and 30 °C, preferably between 23 °C and 27 °C at a flow rate of 100 ml/min.

3. A portable unit (2) according to any of claims 1 and 2, wherein said energy source comprises means (6) for supplying electrical power to heating means (21) arranged externally to the unit, such as within the intravenous tube (3).

4. A portable unit (2) according to claim 3, wherein the said means for supplying electrical power are arranged within or adjacent to the elongated opening (4) of the housing.

5. A portable unit (2) according to any of the preceding claims comprising control means for controlling the operation of at least one element of the unit in response to at least one control input from means for providing a measure of at least one physical property of the flow of intravenous fluid.

6. A portable unit (2) according to claim 5, wherein said means for providing a measure of at least one physical property of the intravenous fluid comprises temperature measure means (10) for measuring the temperature of the flow of fluid in the intravenous tube (3) and providing an output accordingly to the control means.

7. A portable unit (2) according to claim 6, wherein the temperature measuring means (10) are arranged within or adjacent to the elongated opening (4) of the housing.

8. A portable unit (2) according to any of claims 5 to 7, wherein said means for providing a measure of at least one physical property of the intravenous fluid comprises means for providing a measure of the flow rate of fluid in the intravenous tube (3) and providing an input accordingly to the control means.

9. A portable unit (2) according to claim 8, wherein said means for providing a measure of the flow rate of fluid in the intravenous tube (3) comprises alarm means for providing an alarm signal when the source (14) of intravenous fluid is empty.

10. A portable unit (2) according to claim 8 or 9, wherein said means for providing a measure of the flow rate of fluid in the intravenous tube (3) comprises alarm means for providing an alarm signal in case the flow of intravenous fluid is interrupted.

11. A portable unit (2) according to any of claims 5 to 10, wherein the control means controls the operation of the pump (5).

12. A portable unit (2) according to any of claims 5 to 11, wherein the control means controls the supply of power from the energy source to heat intravenous fluid flowing in the intravenous tube (3).

13. A portable unit (2) according to any of claims 5 to 12, wherein the operation of the pump (5) and/or the supply of energy from the energy source to heat intravenous fluid flowing in the intravenous tube (3) are controlled so as to limit the temperature of the flow of intravenous fluid to a temperature interval between 35°C and 40°C such as between 36°C and 38°C.

14. A system (1) for heating and administering a flow of intravenous fluid comprising:
an intravenous tube (3) enclosed in a sterile packaging and having a liquid inlet (8) and a liquid outlet (15) for connecting a source (14) of intravenous fluid to an intravenous catheter (9), said intravenous tube (3) comprising heating means integrated therein for raising the temperature of the intravenous flow in the tube (3),
**characterised in that**
the intravenous tube (3) comprises two one-way valves (16) and a flexible and elastic length of said intravenous tube between the two one-way valves, and connection means for connecting said heating means with an external energy source, and
a portable unit according to any of claims 1 to 13 comprising said external energy source.

15. A system (1) according to claim 14, wherein the energy source is connected to a heating element in the portable unit, which then is in contact with the outer surface of a part of the intravenous tube (3).

16. A system (1) according to claim 15, wherein the heating element in the portable unit during operation is in contact with the outer surface of part of the intravenous tube (3) received in the elongated opening (4) of the portable unit.

17. A system (1) according to any of claims 14 to 16, wherein the surface area in contact with the intravenous fluid of said heating means comprises an area of at least 20 cm², preferably at least 30 cm² and most preferred at least 40 cm².

18. A system (1) according to claim 14 or 17, wherein said integrated heating means comprises one or more heating wires (21) inlaid in the enclosure (22) of the intravenous tube (3).

19. A system (1) according to any of claims 14, 17 and 18, wherein said integrated heating means comprising heating wires (21) encased in the wall (23) of the intravenous tube (3).

20. A system (1) according to claim 18 or 19, wherein said heating means (21) extends a substantial portion of the tube (3) length, preferably between 75 cm and 200 cm of the tube length, preferably between 125 cm and 175 cm of the tube (3) length.

## Patentansprüche

1. Tragbare Einheit (2) zum Bereitstellen von Energie zum Erwärmen von Infusionsfluiden und zum Verabreichen der Fluide, wobei die Einheit ein Gehäuse umfasst, in dem Folgendes angeordnet ist:
eine längliche Öffnung (4) zum Aufnehmen eines Abschnitts eines biegsamen elastischen Infusionsschlauchs (3) zum Anschließen einer Quelle (14) für ein Infusionsfluid an einen Infusionskatheter (9),
ein biegsamer elastischer Infusionsschlauch (3),
eine Energiequelle, die eine oder mehrere wiederaufladbare Batteriezellen umfasst, die dafür eingerichtet sind, elektrische Energie bereitzustellen, um ein Infusionsfluid, das im Infusionsschlauch (3) fließt, zu erwärmen, und
eine Pumpe (5) zum Steuern der Durchflussrate des Infusionsfluids im Infusionsschlauch (3), wobei die Pumpe (5) arbeitet, indem sie auf eine Außenfläche des Abschnitts des biegsamen Infusionsschlauchs (3) einwirkt,
**dadurch gekennzeichnet, dass**
die Pumpe (5) arbeitet, indem sie auf die Außenfläche des biegsamen Abschnitts des Infusionsschlauchs (3), der in der länglichen Öffnung (4) aufgenommen wird, mittels eines Einzelkolbens einwirkt, der während des Betriebs die Außenfläche des biegsamen und elastischen Abschnitts des Infusionsschlauchs (3) zwischen zwei im Infusionsschlauch (3) enthaltenen Rückschlagventilen (16) abwechselnd zusammendrückt und freigibt, und dadurch, dass das Gehäuse außerdem Befestigungsmittel (7, 8) zum Befestigen der Einheit an einem Patienten, der die Infusionsfluide empfängt, umfasst.

2. Tragbare Einheit (2) nach Anspruch 1, wobei die Energiequelle dafür eingerichtet ist, ausreichend Leistung bereitzustellen, um bei einer Durchflussrate von 100 ml/min die Temperatur des Infusionsfluids um einen Betrag zwischen 20°C und 30°C und vorzugsweise zwischen 23°C und 27°C zu erhöhen.

3. Tragbare Einheit (2) nach einem der Ansprüche 1 und 2, wobei die Energiequelle ein Mittel (6) umfasst, um elektrische Energie für ein Heizmittel (21) bereitzustellen, das außerhalb der Einheit angeordnet ist, wie etwa innerhalb des Infusionsschlauchs (3).

4. Tragbare Einheit (2) nach Anspruch 3, wobei das Mittel zum Bereitstellen von elektrischer Energie innerhalb oder benachbart zur länglichen Öffnung (4) des Gehäuses angeordnet ist.

5. Tragbare Einheit (2) nach einem der vorhergehenden Ansprüche, die ein Steuerungsmittel zum Steuern des Betriebs wenigstens eines Elements der Einheit umfasst, und dies in Reaktion auf wenigstens eine Steuerungseingabe von einem Mittel zum Bereitstellen einer Messung wenigstens einer physikalischen Eigenschaft des Flusses des Infusionsfluids.

6. Tragbare Einheit (2) nach Anspruch 5, wobei das Mittel zum Bereitstellen einer Messung wenigstens einer physikalischen Eigenschaft des Infusionsfluids ein Mittel (10) zur Temperaturmessung zum Messen der Temperatur des Fluidflusses im Infusionsschlauch (3) und zum Bereitstellen einer entsprechenden Ausgabe an das Steuerungsmittel umfasst.

7. Tragbare Einheit (2) nach Anspruch 6, wobei das Mittel (10) zur Temperaturmessung innerhalb oder benachbart zur länglichen Öffnung (4) des Gehäuses angeordnet ist.

8. Tragbare Einheit (2) nach einem der Ansprüche 5 bis 7, wobei das Mittel zum Bereitstellen einer Messung wenigstens einer physikalischen Eigenschaft des Infusionsfluids ein Mittel zum Bereitstellen einer Messung der Fluiddurchflussrate im Infusionsschlauch (3) und zum Bereitstellen einer entsprechenden Eingabe an das Steuerungsmittel umfasst.

9. Tragbare Einheit (2) nach Anspruch 8, wobei das Mittel zum Bereitstellen einer Messung der Fluiddurchflussrate im Infusionsschlauch (3) ein Alarmmittel umfasst, um ein Alarmsignal bereitzustellen, falls die Quelle (14) für das Infusionsfluid leer ist.

10. Tragbare Einheit (2) nach Anspruch 8 oder 9, wobei das Mittel zum Bereitstellen einer Messung der Fluiddurchflussrate im Infusionsschlauch (3) ein Alarmmittel umfasst, um ein Alarmsignal bereitzustellen, falls der Fluss des Infusionsfluids unterbrochen ist.

11. Tragbare Einheit (2) nach einem der Ansprüche 5 bis 10, wobei das Steuerungsmittel den Betrieb der Pumpe (5) steuert.

12. Tragbare Einheit (2) nach einem der Ansprüche 5 bis 11, wobei das Steuerungsmittel die Zuführung von Energie von der Energiequelle zum Erwärmen des im Infusionsschlauch (3) fließenden Infusionsfluids steuert.

13. Tragbare Einheit (2) nach einem der Ansprüche 5 bis 12, wobei der Betrieb der Pumpe (5) und/oder die Zuführung von Energie von der Energiequelle zum Erwärmen des im Infusionsschlauch (3) fließenden Infusionsfluids so gesteuert werden, dass die Temperatur des Flusses von Infusionsfluid auf ein Temperaturintervall zwischen 35°C und 40°C, wie etwa zwischen 36°C und 38°C, begrenzt wird.

14. System (1) zum Erwärmen und Verabreichen eines Flusses von Infusionsfluid, Folgendes umfassend:
einen Infusionsschlauch (3), der in einer sterilen Verpackung beigefügt ist und der einen Flüssigkeitseinlass (8) und einen Flüssigkeitsauslass (15) hat, um eine Quelle (14) für ein Infusionsfluid mit einem Infusionskatheter (9) zu verbinden, wobei der Infusionsschlauch (3) ein in ihn integriertes Heizmittel umfasst, um die Temperatur des Infusionsflusses im Schlauch (3) anzuheben,
**dadurch gekennzeichnet, dass**
der Infusionsschlauch (3) Folgendes umfasst: zwei Rückschlagventile (16) und einen biegsamen und elastischen Abschnitt des Infusionsschlauchs zwischen den zwei Rückschlagventilen sowie ein Anschlussmittel, um das Heizmittel an eine externe Energiequelle anzuschließen, und
eine tragbare Einheit nach einem der Ansprüche 1 bis 13, die die externe Energiequelle umfasst.

15. System (1) nach Anspruch 14, wobei die Energiequelle mit einem Heizelement in der tragbaren Einheit verbunden ist, das dann in Kontakt mit der Außenfläche eines Teils des Infusionsschlauchs (3) steht.

16. System (1) nach Anspruch 15, wobei das Heizelement in der tragbaren Einheit während des Betriebs in Kontakt mit der Außenfläche eines Teils des Infusionsschlauchs (3) steht, der in der länglichen Öffnung (4) der tragbaren Einheit aufgenommen wird.

17. System (1) nach einem der Ansprüche 14 bis 16, wobei die mit dem Infusionsfluid in Kontakt stehende Oberfläche des Heizmittels einen Flächeninhalt von wenigstens 20 cm², vorzugsweise wenigstens 30 cm² und noch bevorzugter wenigstens 40 cm² umfasst.

18. System (1) nach Anspruch 14 oder 17, wobei das integrierte Heizmittel einen oder mehrere Heizdrähte (21) umfasst, die in den umhüllten Raum (22) des Infusionsschlauchs (3) eingelegt sind.

19. System (1) nach einem der Ansprüche 14, 17 und 18, wobei das integrierte Heizmittel Heizdrähte (21) umfasst, die in die Wand (23) des Infusionsschlauchs (3) eingebettet sind.

20. System (1) nach Anspruch 18 oder 19, wobei das Heizmittel (21) sich über einen wesentlichen Abschnitt der Länge des Schlauchs (3) erstreckt, vorzugsweise zwischen 75 cm und 200 cm der Schlauchlänge und vorzugsweise zwischen 125 cm und 175 cm der Länge des Schlauchs (3).

## Revendications

1. Unité portable (2) pour la fourniture d'énergie pour chauffer des fluides intraveineux et pour l'administration desdits fluides, l'unité comprenant un logement dans lequel sont agencés
une ouverture allongée (4) pour recevoir une longueur d'un tube intraveineux souple et élastique (3) pour raccorder une source (14) de fluide intraveineux à un cathéter intraveineux (9),
un tube intraveineux souple et élastique (3),
une source d'énergie comprenant une ou plusieurs piles rechargeables agencée pour fournir une puissance électrique pour chauffer le fluide intraveineux s'écoulant dans le tube intraveineux (3), et
une pompe (5) pour réguler le débit du fluide intraveineux dans le tube intraveineux (3), la pompe (5) fonctionnant par manipulation d'une surface extérieure de ladite longueur du tube intraveineux souple (3),
**caractérisée en ce que**
la pompe (5) fonctionne par manipulation de la surface extérieure de ladite longueur souple du tube intraveineux (3) reçue dans ladite ouverture allongée (4) au moyen d'un seul piston qui, durant le fonctionnement, compresse et relâche alternativement la surface extérieure de la longueur souple et élastique du tube intraveineux (3) entre deux valves anti-retour (16) comprises dans le tube intraveineux (3), et **en ce que** le logement comprend en outre un moyen de montage (7, 8) pour fixer l'unité à un patient recevant les fluides intraveineux.

2. Unité portable (2) selon la revendication 1, dans laquelle ladite source d'énergie est capable de fournir une puissance suffisante pour faire augmenter la température du fluide intraveineux d'une valeur entre 20°C et 30°C, de préférence entre 23°C et 27°C à un débit de 100 mL/minute.

3. Unité portable (2) selon l'une quelconque des revendications 1 et 2, dans laquelle ladite source d'énergie comprend un moyen (6) pour fournir une puissance électrique à un moyen de chauffage (21) agencé à l'extérieur de l'unité, par exemple à l'intérieur du tube intraveineux (3).

4. Unité portable (2) selon la revendication 3, dans laquelle ledit moyen de fourniture d'une puissance électrique est agencé à l'intérieur, ou à côté, de l'ouverture allongée (4) du logement.

5. Unité portable (2) selon l'une quelconque des revendications précédentes comprenant un moyen de commande pour commander le fonctionnement d'au moins un élément de l'unité en réponse à au moins une entrée de commande du moyen de fourniture d'une mesure d'au moins une propriété physique de l'écoulement de fluide intraveineux.

6. Unité portable (2) selon la revendication 5, dans laquelle ledit moyen de fourniture d'une mesure d'au moins une propriété physique du fluide intraveineux comprend un moyen de mesure de la température (10) pour mesurer la température de l'écoulement de fluide dans le tube intraveineux (3) et fournir un résultat correspondant au moyen de commande.

7. Unité portable (2) selon la revendication 6, dans laquelle ledit moyen de mesure de la température (10) est agencé à l'intérieur, ou à côté, de l'ouverture allongée (4) du logement.

8. Unité portable (2) selon l'une quelconque des revendications 5 à 7, dans laquelle ledit moyen de fourniture d'une mesure d'au moins une propriété physique du fluide intraveineux comprend un moyen de fourniture d'une mesure du débit de fluide dans le tube intraveineux (3) et la fourniture d'une entrée correspondante au moyen de commande.

9. Unité portable (2) selon la revendication 8, dans laquelle ledit moyen de fourniture d'une mesure du débit de fluide dans le tube intraveineux (3) comprend un moyen d'alarme pour fournir un signal d'alarme lorsque la source (14) de fluide intraveineux est vide.

10. Unité portable (2) selon la revendication 8 ou 9, dans laquelle ledit moyen de fourniture d'une mesure du débit de fluide dans le tube intraveineux (3) comprend un moyen d'alarme pour fournir un signal d'alarme en cas d'interruption de l'écoulement de fluide intraveineux.

11. Unité portable (2) selon l'une quelconque des revendications 5 à 10, dans laquelle le moyen de commande commande le fonctionnement de la pompe (5).

12. Unité portable (2) selon l'une quelconque des revendications 5 à 11, dans laquelle le moyen de commande commande l'alimentation en énergie de la source d'énergie pour chauffer le fluide intraveineux s'écoulant dans le tube intraveineux (3).

13. Unité portable (2) selon l'une quelconque des revendications 5 à 12, dans laquelle le fonctionnement de la pompe (5) et/ou l'alimentation en énergie de la source d'énergie pour chauffer le fluide intraveineux s'écoulant dans le tube intraveineux (3) sont commandés pour limiter la température de l'écoulement du fluide intraveineux à un intervalle de température entre 35°C et 40°C, par exemple entre 36°C et 38°C.

14. Système (1) de chauffage et d'administration d'un écoulement de fluide intraveineux comprenant :
un tube intraveineux (3) enfermé dans un emballage stérile et ayant un orifice d'entrée de liquide (8) et un orifice de sortie de liquide (15) pour raccorder une source (14) de fluide intraveineux à un cathéter intraveineux (9), ledit tube intraveineux (3) comprenant un moyen de chauffage intégré pour faire augmenter la température de l'écoulement intraveineux dans le tube (3),
**caractérisé en ce que**
le tube intraveineux (3) comprend au moins deux valves anti-retour (16) et une longueur souple et élastique dudit tube intraveineux entre les deux valves anti-retour,
et un moyen de raccordement pour raccorder ledit moyen de chauffage à une source d'énergie externe, et
une unité portable selon l'une quelconque des revendications 1 à 13, comprenant ladite source d'énergie externe.

15. Système (1) selon la revendication 14, dans lequel la source d'énergie est raccordée à un élément chauffant dans l'unité portable, qui est alors en contact avec la surface extérieure d'une partie du tube intraveineux (3).

16. Système (1) selon la revendication 15, dans lequel l'élément chauffant dans l'unité portable est, durant le fonctionnement, en contact avec la surface extérieure d'une partie du tube intraveineux (3) reçue dans l'ouverture allongée (4) de l'unité portable.

17. Système (1) selon l'une quelconque des revendications 14 à 16, dans lequel l'aire de surface dudit moyen de chauffage en contact avec le fluide intraveineux comprend une aire d'au moins 20 cm², de préférence d'au moins 30 cm² et idéalement d'au moins 40 cm².

18. Système (1) selon la revendication 14 ou 17, dans lequel ledit moyen de chauffage intégré comprend un ou plusieurs fils chauffants (21) incrustés dans l'enceinte (22) du tube intraveineux (3).

19. Système (1) selon l'une quelconque des revendications 14, 17 et 18, dans lequel ledit moyen de chauffage intégré comprend des fils chauffants (21) enchâssés dans la paroi (23) du tube intraveineux (3).

20. Système (1) selon la revendication 18 ou 19, dans lequel ledit moyen de chauffage (21) s'étend sur une partie sensible de la longueur du tube (3), de préférence entre 75 cm et 200 cm de la longueur du tube, de manière préférée entre 125 cm et 175 cm de la longueur du tube (3).
